# EUROPÄISCHE PATENTANMELDUNG

(11) **EP 3 050 946 A1**
(43) Veröffentlichungstag der Anmeldung: **03.08.2016**
(21) Anmeldenummer: 16151401.3
(22) Anmeldetag: 15.01.2016
(51) Int. Cl.: C11D 1/90, C07C 233/36, B01F 17/00

(54) **BETAINE MIT SPEZIELLER FETTSÄUREKETTENVERTEILUNG**

(30) Priorität: 30.01.2015 EP 15153151
(71) Anmelder: Evonik Degussa GmbH, 45128 Essen (DE)
(72) Erfinder: JAWORSKA-MASLANKA, Maria, Concord, OH 44077 (US); BEGOIHN, Uwe, 45359 Essen (DE); SPRINGER, Oliver, 46485 Wesel (DE); SCHUCH, Dominik, 42781 Haan (DE); KLEIN, Ralf, 42549 Velbert (DE)

(57) **Zusammenfassung**

Gegenstand der Erfindung sind Betaine mit einer speziellen Fettsäurekettenverteilung, die beispielsweise auf Palmkernöl basieren, wobei das Palmkernöl einen erhöhten Gehalt an ungesättigten C18-Fettsäuren enthält.

## Beschreibung

### Gebiet der Erfindung

Gegenstand der Erfindung sind Betaine mit einer speziellen Fettsäurekettenverteilung, die beispielsweise auf Palmkernöl basieren, wobei das Palmkernöl einen erhöhten Gehalt an ungesättigten C18-Fettsäuren enthält.

### Stand der Technik

Zurzeit werden industriell vorwiegend Betaine eingesetzt, die auf Kokosfett basieren. Insbesondere hochkonzentrierte Betaine mit einem hohen Aktivgehalt sind bislang fast ausschließlich als auf Kokosfett-basierende Cocamidopropylbetaine verfügbar. Natives Kokosfett weist einen Ölsäureanteil (C18:1) von maximal 10 Gew.-% bezogen auf alle im Triglycerid enthaltenen Fettsäuren auf; die zur Herstellung der Betaine eingesetzten technischen Fettsäureschnitte weisen somit zwischen 0 bis 10 Gew.-% Ölsäuregehalt auf. Kokosöl unterscheidet sich von Palmkernöl in seiner Fettsäurekettenverteilung, insbesondere im Ölsäuregehalt, der für Kokosöl generell 5 Gew.-% bis 10 Gew.-%, für Palmkernöl generell 10 Gew.-% bis 20 Gew.-% beträgt:

**Tabelle 1: Fettsäurekettenverteilung (Angaben in Gewichtsprozent) von Kokosfett und Palmkernfett auf Basis gaschromatographischer Analysen gemäß Codex Alimentarius der Food and Agriculture Organization of the United Nations (CODEX STAN 210-1999)**

| Fettsäurerest | C8:0 | C10:0 | C12:0 | C14:0 | C16:0 | C18:0 | C18:1 | C18:2 |
|---|---|---|---|---|---|---|---|---|
| Kokosfett | 4,6-10,0 | 5,0-8,0 | 45,1 - 53,2 | 16,8 - 21,0 | 7,5-10,2 | 2,0-4,0 | 5,0-10,0 | 1,0-2,5 |
| Palmkernfett | 2,4-6,4 | 2,6-5,0 | 45,0 - 55,0 | 14,0 - 18,0 | 6,5-10,0 | 1,0-3,0 | 12,0 - 19,0 | 1,0-3,5 |

**Tabelle 2: Fettsäurekettenverteilung (Angaben in Gewichtsprozent) von Kokosfett und Palmkernfett auf Basis gaschromatographischer Analysen gemäß Thieme RÖMPP Online, Georg Thieme Verlag, 2014.**

| Fettsäurerest | C8:0 | C10:0 | C12:0 | C14:0 | C16:0 | C18:0 | C18:1 | C18:2 |
|---|---|---|---|---|---|---|---|---|
| Kokosfett | 5 - 10 | 5 - 8 | 45 - 53 | 17 - 21 | 7 - 10 | 2 - 4 | 5 - 10 | <2 |
| Palmkernfett | 3 | 5 | 47 - 52 | 16 | 6 - 9 | 2 - 3 | 10 - 18 | 1 - 3 |

Bereits in den 80er Jahren wurden entsprechend kommerziell verfügbare Betaine in der DE2926479 offenbart, welche ein Verfahren zur Herstellung von Betainen basierend auf Fettsäuren mit 6 bis 18 Kohlenstoffatomen durch Quaternierung von Fettsäureamiden mit ω-Halogenalkylcarbonsäuren in wässriger Lösung beschreibt. In den Beispielen wird mit Kokosfett gearbeitet.

Die EP1659109 offenbart ein Verfahren zur Herstellung von hochkonzentrierten fliess- und pumpfähigen wässrigen Lösungen von Betainen mit einem Betaingehalt von mindestens 32 Gew.-% durch Quaternierung von tertiären Aminstickstoff enthaltenden Verbindungen mit ω-Halogencarbonsäuren unter Zugabe mizellarer Verdicker. Als Fettsäuren werden gegebenenfalls gehärtete Kokosfettsäure- oder Palmkernfettsäuremischungen vorgeschlagen, in den Beispielen allerdings nur mit Kokosfett gearbeitet.

Die EP0560114 offenbart wässrige flüssige Lösungen eines Betains basierend auf Acylresten von vorzugsweise gehärteten Kokosfettsäuren oder einem Fettsäuregemisch, welches im Mittel der Kokosfettsäure entspricht, wobei die Lösung einen Festkörpergehalt von mindestens 40 Gew.-%, einen pH-Wert von 5 bis 8 und einen Aminoamidgehalt von <= 1 Gew.-% aufweist, gekennzeichnet durch einen Gehalt an 1 bis 3 Gew.-% (bezogen auf Lösung) einer oder mehreren gesättigten Fettsäuren mit im Mittel 8 bis 18 Kohlenstoffatomen oder einer oder mehreren ungesättigten Fettsäuren mit im Mittel 8 bis 24 Kohlenstoffatomen und 0 bis 4 Gew.-% (bezogen auf Lösung) Glycerin. Als Fettsäuren werden gegebenenfalls gehärtete Kokosfettsäure- oder Palmkernfettsäuremischungen vorgeschlagen, wobei gehärtete Fettsäuremischungen bevorzugt sind. In den Beispielen wird nur mit Kokosfett bzw. Fetten gearbeitet, die einen maximalen Ölsäuregehalt von 11 % aufweisen.

Aufgabe der Erfindung war es, neue Betaine bereitzustellen, die gegenüber den bekannten Betainen verbesserte anwendungstechnische Eigenschaften, wie ein gutes Anschäumverhalten und/oder eine verbesserte Verdickbarkeit, aufweisen.

### Beschreibung der Erfindung

Überraschenderweise wurde gefunden, dass auf Palmkernfett basierende Betaine, die sich von einem speziellen Palmkernfett mit einem erhöhten Ölsäuregehalt in der Fettsäurekettenverteilung ableiten, anwendungstechnische Vorteile besitzen.

Gegenstand der vorliegenden Erfindung sind daher Fettsäureamidoalkylbetaine wie in Anspruch 1 beschrieben.
Ein weiterer Gegenstand der Erfindung ist die Verwendung der erfindungsgemäßen Fettsäureamidoalkylbetaine in kosmetischen, pharmazeutischen und technischen Anwendungen.

Ein Vorteil der vorliegenden Erfindung ist die verbesserte Verdickbarkeit von insbesondere wässrigen Tensidformulierungen. Demgemäß können in Gegenwart der erfindungsgemäßen Betaine höhrere Viskositäten in wässrigen Tensidformulierungen mit analoger Zusammensetzung erreicht werden als mit nicht erfindungsgemäßen Betainen. Weiterhin kann beim Austausch der nicht erfindungsgemäßen Betaine durch erfindungsgemäße Betaine auch die Menge des zugesetzten Verdickers oder Kochsalzes in der Formulierung ohne Viskositätsverlust reduziert werden. Alternativ dazu kann bei gleichbleibender Verdickerkonzentration auch die Menge des erfindungsgemäßen Betains ohne Viskositätsverlust reduziert werden. Demgemäß ermöglichen die erfindungsgemäßen Betaine also generell eine Reduzierung der Gesamtkonzentration einer kosmetischen Tensidformulierung, wodurch sich eine größere Effizienz ergibt. Darüber hinaus wirken sich reduzierte Salzgehalte vorteilhalt auf die Mildheit, das Hautgefühl und die Korrosivität von Tensidformulierungen aus.
Ein weiterer Vorteil der erfindungsgemäßen Betaine ist eine verstärkte und schnellere Schaumentwicklung unter Rühren, Schütteln oder jeglicher anderen Scherkrafteinwirkung in wässriger Lösung im Vergleich zu nicht erfindungsgemäßen Betainen. Eine verstärkte Schaumbildung geht beim Anwender typischerweise mit einer Reduzierung der verwendeten Menge und damit Ressourceneinsparung einher.
Noch ein Vorteil der erfindungsgemäßen Betaine ist die Reduzierung der Steifheit nach Applikation auf Textilgewebe im Vergleich zu nicht erfindungsgemäßen Betainen. Ein weiterer Vorteil der erfindungsgemäßen Betaine ist die Erhöhung der Weichheit nach Applikation auf Textilgewebe im Vergleich zu nicht erfindungsgemäßen Betainen.

Gegenstand der vorliegenden Erfindung sind somit Fettsäureamidoalkylbetaine der allgemeinen Formel I) 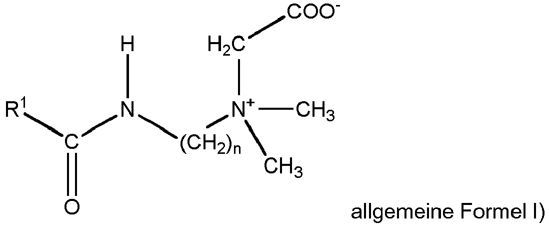 mit
n = 1 bis 10, bevorzugt 2 bis 5, insbesondere 3 und
R¹CO = Mischung von Acylresten eines Palmkernöls,
dadurch gekennzeichnet, dass die Mischung der Acylreste einen Gehalt an Ölsäureacylresten bezogen auf alle Acylreste der Mischung von 12 Gew.-% bis 21 Gew.-%, bevorzugt von 13 Gew.-% bis 17 Gew.-%, besonders bevorzugt von 14 Gew.-% bis 15 Gew.-% aufweist.

Es wird somit vorliegend eine Mischung von Fettsäureamidoalkylbetainen beansprucht.

Unter dem Begriff "Mischung von Acylresten eines Palmkernöls" wird im Zusammenhang mit der vorliegenden Erfindung verstanden, dass diese Mischung die folgend gelistete Menge an jeweiligem Acylrest, angegeben in Gewichtsprozent, enthält, wobei sich die genannten Gewichtsprozente auf alle in der Mischung enthaltene Acylreste beziehen:

| C8:0 | C10:0 | C12:0 | C14:0 | C16:0 | C18:0 | C18:1 | C18:2 |
|---|---|---|---|---|---|---|---|
| 1,0 - 6,4 | 2,0 - 5,0 | 40,0 - 55,0 | 14,0 - 18,0 | 6,0 - 12,0 | 1,0 - 4,0 | 10,0 - 21,0 | 1,0 - 4,0 |

Der Begriff "Mischung von Acylresten eines Palmkernöls" umfasst somit in diesem Zusammenhang auch Mischungen von Acylresten, die originär nicht aus Palmkernöl hergestellt wurden, sondern beispielsweise durch Mischung synthetischer Verbindungen auf die vorgenannte Acylrestverteilung eingestellt wurden.
Der jeweilige Acylrestgehalt lässt sich experimentell mittels einer Kombination zweier flüssigchromatographischer Methoden bestimmen. Dabei werden die einzelnen Betaine der Mischung, die 8 bis 18 Kohlenstoffatome im Acylrest enthalten, zunächst mittels HPLC/RI nach einer von R. Gerhards et.al. beschriebenen Methode (Modern methods for the analysis of cocamidopropyl betaines; Gerhards. R et. Al. ; Tenside, Surfactants, Detergents; 1996 33(1):1-12) aufgetrennt. Da diese Methode eine unzureichende Peak-Separation für die Betaine mit 16 bis 18 Kohlenstoffatomen (C16, C18:0, C18:1 und C18:2) im Acylrest liefert, wenn Oleylamidopropylbetainanteile vorhanden sind, wird dieser Anteil mittels einer zweiten Gradienten-HPLC-Methode aufgetrennt. Dies erfolgt an einer RP-Säule (reversed phase column, Hypersil Gold 100 mm * 3 mm; 5µ, Thermofisher) bei 50 °C in einem Gradienten von 0,05 %iger wässriger Ammoniumformiat-Lösung (pH auf 4.2 mit Ameisensäure eingestellt) und Acetonitril (Gradienten-Programm: Start mit 10 % Acetonitril wird für 1 Minute gehalten; dann 15 minütiger linearer Gradient von 10 % auf 95 % Acetonitril; dann wird 10 Minuten bei 95 % Acetonitril gehalten), wobei die einzelnen Betainfraktionen per CAD (CAD = charged aerosol detector, Dionex Corona Ultra RS, Thermo Scientific) detektiert werden.
Die Acylrestgehalte der einzelnen Betaine, deren Acylreste 8 bis 14 Kohlenstoffatome enthalten sowie die Summe der Acylrestgehalte der Betaine, deren Acylreste 16 bis 18 (C16, C18:0, C18:1 und C18:2) Kohlenstoffatome enthalten, lassen sich folglich aus den Peakflächenprozenten der HPLC/RI-Messung ermitteln.
Die Acylrestgehalte der einzelnen Betaine, deren Acylreste 16 bis 18 (C16, C18:0, C18:1 und C18:2) Kohlenstoffatome enthalten, ergeben sich dann aus deren Peakflächenprozenten aus der HPLC/CAD-Messung nach Normierung auf die Summe der Peakflächenprozente dieser vier Betaine aus der HPLC/RI-Messung. Dazu wird der Peakflächenprozentwert des jeweiligen Betains aus der HPLC/CAD-Messung mit der Summe der Peakflächenprozente dieser vier Betaine aus der HPLC/RI Messung multipliziert und dann durch die Summe der Peakflächenprozente dieser vier Betaine aus der HPLC/CAD-Messung dividiert.
Der Begriff "Acylrestgehalt" ist immer definiert als der Gewichtsanteil bezogen auf alle vorliegenden Acylreste.
Die Begriffe "Kokosfett" und Kokosöl" werden synonym verwendet.

Die Begriffe "Palmkernfett" und "Palmkernöl" werden synonym verwendet.
Alle angegebenen Prozent (%) sind, wenn nicht anders angegeben, Massenprozent.

In einer alternativen, bevorzugten Ausführungsform sind die erfindungsgemäßen Fettsäureamidoalkylbetaine dadurch gekennzeichnet, dass die Acylreste der Mischung einen Gehalt an Ölsäureacylresten bezogen auf alle Acylreste der Mischung von 19 Gew.-% bis 21 Gew.-% aufweisen

Erfindungsgemäß bevorzugte Fettsäureamidoalkylbetaine sind dadurch gekennzeichnet, dass die Mischung von Acylresten des Palmkernöls einen Gehalt an Linolsäureacylresten (C18:2) bezogen auf alle Acylreste der Mischung von 1,5 Gew.-% bis 4 Gew.-%, bevorzugt von 2 Gew.-% bis 3,5 Gew.-%, besonders bevorzugt von 2,5 Gew.-% bis 3 Gew.-% aufweist.

Erfindungsgemäß besonders bevorzugte Fettsäureamidoalkylbetaine sind dadurch gekennzeichnet, dass die Mischung von Acylresten des Palmkernöls
einen Gehalt an Ölsäureacylresten bezogen auf alle Acylreste der Mischung von 12 Gew.-% bis 21 Gew.-%,
und einen Gehalt an Linolsäureacylresten bezogen auf alle Acylreste der Mischung von 2 Gew.-% bis 4 Gew.-%,
insbesondere einen Gehalt an Ölsäureacylresten bezogen auf alle Acylreste der Mischung von 13 Gew.-% bis 21 Gew.-%,
und einen Gehalt an Linolsäureacylresten bezogen auf alle Acylreste der Mischung von 2 Gew.-% bis 4 Gew.-%,
aufweist.

Es ist erfindungsgemäß bevorzugt, dass die Fettsäureamidoalkylbetaine der vorliegenden Erfindung in einer wässrigen Lösung enthalten sind. Gegenstand der vorliegenden Erfindung ist somit eine wässrige Lösung enthaltend die erfindungsgemäßen Fettsäureamidoalkylbetaine. Besonders bevorzugt sind die Fettsäureamidoalkylbetaine vollständig und klar in der wässrigen Lösung gelöst.
Unter dem Begriff "wässrige Lösung" im Zusammenhang mit der vorliegenden Erfindung sind Zusammensetzungen mit einem Wassergehalt von mindestens 10 Gew.-% bezogen auf die Gesamtzusammensetzung zu verstehen.
Die wässrige Lösung ist erfindungsgemäß bevorzugt bei 25 °C und 1 bar Druck flüssig. Insbesondere bevorzugt weist sie bei 25 °C eine Viskosität in einem Bereich von 1 bis 9999 mPa s auf, wobei die Viskosität mit einem Rheometer von Anton Paar, Modell MCR 301, Platte - Platte (40 mm) Geometrie bei einer Temperatur von 25°C im Scherraten-Bereich von 0.1 s-1 bis 1000 s-1 bestimmt wird.

Die erfindungsgemäße wässrige Lösung enthält die erfindungsgemäßen Fettsäureamidoalkylbetaine bevorzugt bezogen auf die gesamte wässrige Lösung in einer Menge von 15 Gew.-% bis 55 Gew.-%, bevorzugt von 21 Gew.-% bis 49 Gew.-%, besonders bevorzugt von 31 Gew.-% bis 39 Gew.-%.
Hierdurch können beispielsweise bestimmte konzentrierte Geschirrspülmittel nur unter Verwendung vergleichsweise hochkonzentrierter Betaine hergestellt werden, da sonst der Wassergehalt der Geschirrspülformulierung zu hoch und der Anteil an Aktivstoffen zu gering ist. Der Betaingehalt wird entweder als Differenz von 100 % und der Summe aus den prozentualen Gehalten an Wasser, Salz und Glycerin berechnet oder alternativ als Differenz des prozentualen Trockenrückstandgehaltes und der Summe der prozentualen Gehalte an Salz und Glycerin. Die Gehaltsbestimmungen erfolgen fachmännisch, beispielsweise in Anlehnung an Oil and Soap, 22, 115, (1945) und J.A.O.A.C., 26, 99, (1943) und Proc.Sci.Sect. Toilet Goods Ass'n, 5, (1946) sowie DIN 51777 und DGF E-III 10 und DGF C-III 13a, sowie DGF H-III 9, sowie DGF B-II 3 / C-III 12.

Erfindungsgemäß bevorzugt weist die wässrige Lösung enthaltend die erfindungsgemäßen Fettsäureamidoalkylbetaine einen pH-Wert in einem Bereich von 3,1 bis 12,9, bevorzugt 3,6 bis 7,9, besonders bevorzugt 4,1 bis 6,9 auf.
Der "pH-Wert" im Zusammenhang mit der vorliegenden Erfindung ist definiert als der Wert, welcher für bei 25 °C nach fünf Minuten Rühren mit einer gemäß ISO 4319 (1977) kalibrierten pH-Elektrode gemessen wird.

Erfindungsgemäß besonders bevorzugt sind wässrige Lösungen enthaltend erfindungsgemäße Fettsäureamidoalkylbetaine mit n=3 in einer Konzentration bezogen auf die Gesamtlösung von 15 Gew.-% bis 55 Gew.-%, bevorzugt 21 Gew.-% bis 49 Gew.-%, besonders bevorzugt 31 Gew.-% bis 39 Gew.-%, dadurch gekennzeichnet, dass die Mischung von Acylresten des Palmkernöls einen Gehalt an Ölsäureacylresten bezogen auf alle Acylreste der Mischung von 12 Gew.-% bis 21 Gew.-%, bevorzugt von 13 Gew.-% bis 17 Gew.-%, besonders bevorzugt von 14 Gew.-% bis 15 Gew.-% aufweist,
und insbesondere bevorzugt die Mischung von Acylresten des Palmkernöls einen Gehalt an Linolsäureacylresten bezogen auf alle Acylreste der Mischung von 2 Gew.-% bis 4 Gew.-% aufweist.

Erfindungsgemäß ganz besonders bevorzugt sind wässrige Lösungen enthaltend erfindungsgemäße Fettsäureamidoalkylbetaine mit n=3 in einer Konzentration bezogen auf die Gesamtlösung von 15 Gew.-% bis 55 Gew.-%, besonders bevorzugt 31 Gew.-% bis 39 Gew.-%, dadurch gekennzeichnet, dass die Mischung von Acylresten des Palmkernöls einen Gehalt an Ölsäureacylresten bezogen auf alle Acylreste der Mischung von 13 Gew.-% bis 21 Gew.-%, aufweist,
und insbesondere bevorzugt die Mischung von Acylresten des Palmkernöls einen Gehalt an Linolsäureacylresten bezogen auf alle Acylreste der Mischung von 2 Gew.-% bis 4 Gew.-% aufweist.

Die erfindungsgemäßen Fettsäureamidoalkylbetaine lassen sich nach Verfahren herstellen, wie beispielsweise in der DE2926479 oder DE4207386 C1 beschrieben.
Um den Fettsäureamidoalkylbetaingehalt in einer wässrigen Lösung entsprechend zu erhöhen, kann Wasser beispielsweise durch Abdampfen entfernt werden.
Herstellungsbedingt können sowohl die Amidamine als auch die Betaine einen geringen Restgehalt an freien Fettsäuren enthalten. Dieser ist in den Amidaminen als Säurezahl und in den Betainen als freie Fettsäure bestimmbar, beispielsweise mittels HPLC-Analyse gemäß M. J. Cooper, M. W. Anders. Anal. Chem. , 1974, 46 (12), pp 1849-1852.
Erfindungsgemäß bevorzugt enthält die wässrige Lösung freie Fettsäuren, insbesondere Fettsäuren von Palmkernöl. Die freien Fettsäuren sind bezogen auf die gesamte Lösung bevorzugt in einer Menge von 0,05 Gew.-% bis 2 Gew.-%, besonders bevorzugt von 0,1 Gew.-% bis 1,5 Gew.-% enthalten.
Zusätzlich kann es vorteilhaft sein und ist daher bevorzugt, wenn die erfindungsgemäßen Lösungen Glycerin enthalten, insbesondere in einer Menge von 0,05 Gew.-% bis 5 Gew.-%, besonders bevorzugt von 1,0 Gew.-% bis 3,0 Gew.-%.

Die erfindungsgemäßen Fettsäureamidoalkylbetaine lassen sich vorteilhaft zur Herstellung von wässrigen Formulierungen, bevorzugt Pflege- und Reinigungsformulierungen, insbesondere kosmetischen und dermatologischen Formulierungen, verwenden. Somit sind Formulierungen, insbesondere kosmetische und dermatologische Formulierungen, enthaltend die erfindungsgemäßen Fettsäureamidoalkylbetaine ebenfalls Gegenstand der vorliegenden Erfindung.
Erfindungsgemäß bevorzugte Formulierungen enthalten zusätzlich mindestens ein weiteres Tensid, insbesondere Sodium Laureth Sulfate. Solche Formulierungen sind beispielsweise Shampoos, Duschbäder, Badeöle, Flüssigseifen, Mundspülungen, Haushaltsreiniger, Industriereiniger, Geschirrspülmittel, Textilpflegemittel und Textilveredelungsmittel.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele:

### Beispiel 1: Amidamine

Als Vorstufen der Betaine wurden zunächst die Amidamine durch Reaktion von Dimethylaminopropylamin mit den entsprechenden Fettsäure-basierten Triglyceriden in bekannter Art und Weise gemäß EP656346 hergestellt. Als Triglyceride wurden gehärtetes und ungehärtetes Kokosfett sowie ungehärtetes Palmkernfett und Mischungen der zuvor genannten Rohstoffe eingesetzt. Nach Syntheseende wiesen alle Amidamine eine Säurezahl und eine Esterzahl von maximal 2 mg KOH / g auf:

**Amidamin A wurde aus einem Triglycerid mit folgender Fettsäurekettenverteilung hergestellt:**

| | |
|---|---|
| C8 | 6,6 % |
| C10 | 5,6 % |
| C12 | 46,4 % |
| C14 | 18,7 % |
| C16 | 9,9 % |
| C18:0 | 11,8 % |
| **C18:1** | **0,2 %** |
| andere | 0,8 % |

**Amidamin B wurde aus einem Triglycerid mit folgender Fettsäurekettenverteilung hergestellt:**

| | |
|---|---|
| C8 | 7,5 % |
| C10 | 6,1 % |
| C12 | 47,5 % |
| C14 | 18,8 % |
| C16 | 9,2 % |
| C18:0 | 2,8 % |
| **C18:1** | 6,1 % |
| C18:2 | 1,5 % |
| andere | 0,5 % |

**Amidamin C wurde aus einem Triglycerid mit folgender Fettsäurekettenverteilung hergestellt:**

| | |
|---|---|
| C8 | 5,6 % |
| C10 | 4,9 % |
| C12 | 47,0 % |
| C14 | 17,1 % |
| C16 | 9,6 % |
| C18:0 | 2,6 % |
| **C18:1** | **11,0 %** |
| C18:2 | 2,1 % |
| andere | 0,1 % |

**Amidamin D wurde aus einem Triglycerid mit folgender Fettsäurekettenverteilung hergestellt:**

| | |
|---|---|
| C8 | 4,3 % |
| C10 | 3,9 % |
| C12 | 48,1 % |
| C14 | 16,7 % |
| C16 | 8,6 % |
| C18:0 | 2,5 % |
| **C18:1** | **13,5 %** |
| C18:2 | 2,3 % |
| andere | 0,1 % |

**Amidamin E wurde aus einem Triglycerid mit folgender Fettsäurekettenverteilung hergestellt:**

| | |
|---|---|
| C8 | 3,4 % |
| C10 | 3,3 % |
| C12 | 47,3 % |
| C14 | 16,2 % |
| C16 | 9,1 % |
| C18:0 | 2,2 % |
| **C18:1** | **15,8 %** |
| C18:2 | 2,6 % |
| andere | 0,1 % |

**Amidamin F wurde aus einem Triglycerid mit folgender Fettsäurekettenverteilung hergestellt:**

| | |
|---|---|
| C8 | 1,4 % |
| C10 | 2,5 % |
| C12 | 43,0 % |
| C14 | 15,9 % |
| C16 | 10,9 % |
| C18:0 | 2,8 % |
| **C18:1** | **19,8 %** |
| C18:2 | 3,2 % |
| andere | 0,5 % |

### Beispiel 2: Betaine (nicht erfindungsgemäß)

Synthese von Betain A enthaltend 0,2 % Ölsäurereste bezogen auf alle Acylreste: Gemäß EP0560114 wurde Amidamin A mit Natriummonochloracetat zu Betain A umgesetzt, bis ein Amidamingehalt von 0,5 % unterschritten wurde. Das Produkt wies einen Trockenrückstand von 45,8 %, einen NaCl-Gehalt von 6,4 % und einen Glyceringehalt von 2,6 % auf, wodurch ein Betaingehalt von 36,8 % berechnet wurde.

Synthese von Betain B enthaltend 6,0 % Ölsäurereste bezogen auf alle Acylreste: Gemäß EP0560114 wurde Amidamin B mit Natriummonochloracetat zu Betain B umgesetzt, bis ein Amidamingehalt von 0,5 % unterschritten wurde. Das Produkt wies einen Trockenrückstand von 45,8 %, einen NaCl-Gehalt von 6,8 % und einen Glyceringehalt von 2,6 % auf, wodurch ein Betaingehalt von 36,4 % berechnet wurde.

Synthese von Betain C enthaltend 11,0 % Ölsäurereste bezogen auf alle Acylreste: Gemäß EP0560114 wurde Amidamin C mit Natriummonochloracetat zu Betain C umgesetzt, bis ein Amidamingehalt von 0,5 % unterschritten wurde. Das Produkt wies einen Trockenrückstand von 45,5 %, einen NaCl-Gehalt von 7,4 % und einen Glyceringehalt von 2,5 % auf, wodurch ein Betaingehalt von 35,6 % berechnet wurde.

### Beispiel 3: Betaine (erfindungsgemäß)

Synthese von Betain D enthaltend 13,5 % Ölsäurereste bezogen auf alle Acylreste:
Aufgrund des vergleichsweise hohen C18-Anteils und einer daraus bekannterweise resultierenden starken Viskositätserhöhung während der Synthese in etwa 45 %iger wässriger Lösung wurde Amidamin D mit Natriummonochloracetat zunächst zu einem Betain D* mit einen Trockenrückstand von 37 % umgesetzt, bis ein Amidamingehalt von 0,4 % unterschritten wurde. Anschließend wurde Betain D hergestellt, indem so viel Wasser abgedampft wurde, dass eine Lösung mit einem Trockengewichtsgehalt von 45,1 %, einem NaCl-Gehalt von 7,8 % und einem Glyceringehalt von 2,4 % erhalten wurde, wodurch sich ein Betaingehalt bezogen auf die Gesamtlösung von 34,9 % ergibt.

Synthese von Betain E enthaltend 15,8 % Ölsäurereste bezogen auf alle Acylreste:
Aufgrund des vergleichsweise hohen C18-Anteils und einer daraus bekannterweise resultierenden starken Viskositätserhöhung während der Synthese in etwa 45 %iger wässriger Lösung wurde Amidamin E mit Natriummonochloracetat zunächst zu einem Betain E* mit einen Trockenrückstand von 37 % umgesetzt, bis ein Amidamingehalt von 0,4 % unterschritten wurde. Anschließend wurde Betain E hergestellt, indem so viel Wasser abgedampft wurde, dass eine Lösung mit einem Trockengewichtsgehalt von 44,3 %, einem NaCl-Gehalt von 7,6 % und einem Glyceringehalt von 2,3 % erhalten wurde, wodurch sich ein Betaingehalt bezogen auf die Gesamtlösung von 34,4 % ergibt.

Synthese von Betain F enthaltend 19,8 % Ölsäurereste bezogen auf alle Acylreste:
Aufgrund des vergleichsweise hohen C18-Anteils und einer daraus bekannterweise resultierenden starken Viskositätserhöhung während der Synthese in etwa 45 %iger wässriger Lösung wurde Amidamin F mit Natriummonochloracetat zunächst zu einem Betain F* mit einen Trockenrückstand von 37 % umgesetzt bis ein Amidamingehalt von 0,4 % unterschritten wurde. Anschließend wurde Betain F hergestellt, indem so viel Wasser abgedampft wurde, dass eine Lösung mit einem Trockengewichtsgehalt von 45,0 %, einem NaCl-Gehalt von 7,3 %
   und einem Glyceringehalt von 2,4 % erhalten wurde, wodurch sich ein Betaingehalt bezogen auf die Gesamtlösung von 35,3 % ergibt.

Für alle Betaine A bis F wurde nach der Synthese bestätigt, dass ihre Fettsäurekettenverteilung der entspricht, wie die der oben eingesetzten Triglyceride.

### Beispiel 4: Verdickbarkeit von kosmetischen Formulierungen

Die Verdickbarkeit wurde in einem sehr gängigen, marktüblichen Tensidsystem getestet. Die Formulierungsbestandteile sind in den Zusammensetzungen in Form der allgemein anerkannten INCI-Nomenklatur unter Verwendung der englischen Begriffe benannt. Alle Konzentrationen in den Anwendungsbeispielen sind in Gewichtsprozent angegeben. Die Viskositätsbestimmung erfolgte mittels Brookfield-Viskosimeter (Brookfield LVF, Spindel 2, 30 UpM) bei 25 °C.

Die Daten in Tabelle 3 und 4 belegen, dass Formulierungen, die sich ausschließlich durch das eingesetzte Betain unterscheiden, eine deutlich höhere Viskosität aufweisen, wenn statt der nicht erfindungsgemäßen Betaine A oder B die erfindungsgemäßen Betaine E oder F eingesetzt werden. Die Daten in Tabelle 5 zeigen, dass eine vorgegebene Formulierungsviskosität bereits durch Zugabe geringerer Mengen der erfindungsgemäßen Betaine erreicht wird als durch Zugabe von nicht erfindungsgemäßen Betainen.

**Tabelle 3: Viskositätsvergleich in einer Standard-Tensidformulierung**

| | | | | |
|---|---|---|---|---|
| Texapon® NSO, BASF SE, 28%-ig (INCI: Sodium Laureth Sulfate) | 32,0 % | 32,0 % | 32,0 % | 32,0 % |
| Betain A | 8,0 % | | | |
| Betain B | | 8,0 % | | |
| Betain E | | | 8,0 % | |
| Betain F | | | | 8,0 % |
| ANTIL® 171, Evonik Industries AG (INCI: PEG-18 Glyceryl Cocoate/Oleate) | 2,5 % | 2,5 % | 2,5 % | 2,5 % |
| Natriumchlorid | 0,5 % | 0,5 % | 0,5 % | 0,5 % |
| Wasser | ad 100% | ad 100% | ad 100% | ad 100% |
| Citric Acid, 30% | ad pH 5,5 | ad pH 5,5 | ad pH 5,5 | ad pH 5,5 |
| Viskosität [mPa s] | 3115 | 4395 | 6677 | 8152 |

**Tabelle 4: Viskositätsvergleich in Abhängigkeit der Salzkonzentration in einer wässrigen Formulierung aus 32 % Texapon® NSO (BASF SE, 28%-ig) und 8 % Betain**

| Salzgehalt der Formulierung | 1 % NaCl | 2 % NaCl | 3% NaCl | 4 % NaCl |
|---|---|---|---|---|
| Viskosität [mPa s] mit Betain A | 32 | 5226 | 6528 | 24930 |
| Viskosität [mPa s] mit Betain E | 533 | 6218 | 45820 | 96530 |

**Tabelle 5: Erforderliche Betaingehalte für eine Formulierungsviskosität von 3500 mPa s**

| | | | | |
|---|---|---|---|---|
| Texapon® NSO, BASF SE, 28%-ig (INCI: Sodium Laureth Sulfate) | 32,0 % | 32,0 % | | |
| Betain A | 8,0 % | | 12,8 % | |
| Betain E | | 6,9 % | | 6,7 % |
| REWOTERIC® AM C, Evonik Industries AG (INCI: Sodium Cocoamphoacetate) | | | 15,0 % | 15,0 % |
| REWOPOL® SB F 12 P, Evonik Industries AG (INCI: Disodium Lauryl Sulfosuccinate) | | | 3,6 % | 3,6 % |
| ANTIL® 171, Evonik Industries AG (INCI: PEG-18 Glyceryl Cocoate/Oleate) | 2,5 % | 2,5 % | 3,8 % | 3,8 % |
| Natriumchlorid | 0,5% | 0,5% | | |
| Wasser | ad 100% | ad 100% | ad 100% | ad 100% |
| Citric Acid, 30% | ad pH 5,5 | ad pH 5,5 | ad pH 5,5 | ad pH 5,5 |
| Viskosität [mPa s] | 3500 | 3500 | 3500 | 3500 |

### Beispiel 5: Schaumverhalten

In SITA-Messungen (c = 0,5 %, T = 30 °C, Wasser mit 10 °dH (Deutsche Härte), pH = 6, 1500 UpM) zeigten die erfindungsgemäßen Betaine D und E gegenüber den nicht erfindungsgemäßen Betainen A und C ein deutlich besseres Anschäumverhalten. Die Ergebnisse sind in Tabelle 6 dargestellt.

**Tabelle 6: Anschäumverhalten in 0,5 %iger wässriger Lösung**

| | | | | | | |
|---|---|---|---|---|---|---|
| Rührdauer [s] | 10 | 20 | 30 | 40 | 50 | 60 |
| Schaumhöhe Betain A [mm] | 330 | 507 | 763 | 973 | 1022 | 1044 |
| Schaumhöhe Betain C [mm] | 356 | 530 | 807 | 976 | 1042 | 1061 |
| Schaumhöhe Betain D [mm] | 494 | 771 | 941 | 1019 | 1051 | 1067 |
| Schaumhöhe Betain E [mm] | 591 | 1031 | 1031 | 1039 | 1047 | 1054 |

### Beispiel 6: Weichheit und Steifheit nach Betainapplikation auf Textilgewebe

Zum Vergleich von Weichheit und Steifheit wurden die Betaine mittels Foulard auf Wirkware der Firma wfk-Testgewebe GmbH appliziert (Walzenauftrag, Typ HVF, Mathis AG, Auftrag aus einer 0,25%igen (aktiv) Flotte = 0,003 g/g für die Weichheitsmessungen, Trocknung der Gewebe im Labor-Trockner (Typ LTE, Mathis AG) bei 105°C für 2 Minuten plus Verweilzeit und Fixierung bei 150°C für 3 Minuten ohne Verweilzeit, Lagerung der Textilien für ca. 24 Stunden im Klimaraum bei 23°C und 50% Luftfeuchte) und dann Messungen mittels Tissue Softness Analyzer der Fa. Emtec Electronic GmbH durchgeführt. In Tabelle 7 sind die Ergebnisse als Mittelwerte aus 5 Einzelmessungen dargestellt. Im Falle des Parameters Weichheit erwies sich die Applikation des erfindungsgemäßen Betains D als vorteilhaft im Vergleich zu den nicht erfindungsgemäßen Betainen A und C, da niedrigere Werte eine erhöhte Weichheit bedeuten. Im Falle des Parameters Steifheit zeigten höhere Werte eine reduzierte Steifheit an, wodurch sich auch hier die Applikation des erfindungsgemäßen Betains D als vorteilhaft gegenüber den nicht erfindungsgemäßen Betainen A und C erwies.

**Tabelle 7: TSA-Messergebnisse**

| | Weichheit | Steifheit [mm/N] |
|---|---|---|
| Betain A [mm] | 11,4 | 2,75 |
| Betain C [mm] | 11,4 | 2,78 |
| Betain D [mm] | 10,7 | 2,93 |

### Beispiel 7: Herstellung hochkonzentrierter Geschirrspülmittel

Wie aus Tabelle 8 hervorgeht, gelang die Herstellung eines hochkonzentrierten Geschirrspülmittels in Anlehnung an DE102007005942 nur unter Verwendung des vergleichsweise hochkonzentrierten Betains D im Gegensatz zum niedriger konzentrierten Betain D*, wenn, wie im vorliegenden Beispiel, mit 28 %iger wässriger Natriumlaurylethersulfat-Lösung gearbeitet wird, die aufgrund der niedrigen Viskosität und Kaltverarbeitbarkeit den Marktstandard darstellt.

**Tabelle 8: Herstellung hochkonzentrierter Geschirrspülmittel mit einem pH-Wert von 6**

| Rohstoff | Rohstoff-konzentration | Gewünschter Aktivstoff- / Betaingehalt in Formulierung | Rohstoff-menge | Rohstoff-menge |
|---|---|---|---|---|
| Texapon® NSO | 28,0 % | 21,0 % | 75,0 % | 75,0 % |
| Betain D* | 37.0 % (28,6 % Betain) | 7,0 % | 24,5 % | |
| Betain D | 45,1 % (34,9 % Betain) | 7,0 % | | 20,0 % |
| Ethanol | 99,8 % | 5,0 % | 5,0 % | 5,0 % |
| Summe | | | 104,5 % | 100 % |

## Patentansprüche

1. Fettsäureamidoalkylbetain der allgemeinen Formel I) 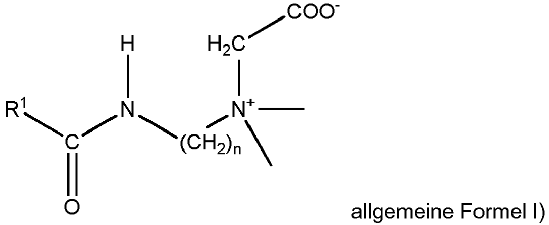 mit
n = 1 bis 10, bevorzugt 2 bis 5, insbesondere 3 und
R¹CO = Mischung von Acylresten eines Palmkernöls,
**dadurch gekennzeichnet, dass** die Mischung der Acylreste einen Gehalt an Ölsäureacylresten bezogen auf alle Acylreste der Mischung von 12 Gew.-% bis 21 Gew.-%, bevorzugt von 13 Gew.-% bis 17 Gew.-%, besonders bevorzugt von 14 Gew.-% bis 15 Gew.-% aufweist.

2. Fettsäureamidoalkylbetain gemäß Anspruch 1, **dadurch gekennzeichnet, dass** die Mischung von Acylresten des Palmkernöls einen Gehalt an Linolsäureacylresten (C18:2) bezogen auf alle Acylreste der Mischung von 1,5 Gew.-% bis 4 Gew.-%, bevorzugt von 2 Gew.-% bis 3,5 Gew.-%, besonders bevorzugt von 2,5 Gew.-% bis 3 Gew.-% aufweist.

3. Fettsäuremidoalkylbetain gemäß Anspruch 1 oder 2, **dadurch gekennzeichnet, dass** die Mischung von Acylresten des Palmkernöls
einen Gehalt an Ölsäureacylresten bezogen auf alle Acylreste der Mischung von 12 Gew.-% bis 21 Gew.-%,
und einen Gehalt an Linolsäureacylresten bezogen auf alle Acylreste der Mischung von 2 Gew.-% bis 4 Gew.-%
aufweist.

4. Wässrige Lösung enthaltend Fettsäureamidoalkylbetaine gemäß mindestens einem der vorherigen Ansprüche.

5. Wässrige Lösung gemäß Anspruch 4, **dadurch gekennzeichnet, dass** sie die Fettsäureamidoalkylbetaine in einer Menge von 15 Gew.-% bis 55 Gew.-%, bevorzugt von 21 Gew.-% bis 49 Gew.-%, besonders bevorzugt von 31 Gew.-% bis 39 Gew.-%, enthält.

6. Wässrige Lösung gemäß Anspruch 4 oder 5, **dadurch gekennzeichnet, dass** sie einen pH-Wert in einem Bereich von 3,1 bis 12,9, bevorzugt 3,6 bis 7,9, besonders bevorzugt 4,1 bis 6,9 aufweist.

7. Wässrige Lösung gemäß mindestens einem der Ansprüche 4 bis 6, **dadurch gekennzeichnet, dass** sie freie Fettsäuren enthält.

8. Wässrige Lösung gemäß mindestens einem der Ansprüche 4 bis 7, **dadurch gekennzeichnet, dass** sie Glycerin enthält.

9. Wässrige Lösung gemäß mindestens einem der Ansprüche 4 bis 8 enthaltend ein weiteres Tensid.
